(19) Europäisches Patentamt
European Patent Office
Office européen des brevets

(11) Veröffentlichungsnummer: **0 432 309 B1**

(12) # EUROPÄISCHE PATENTSCHRIFT

(45) Veröffentlichungstag der Patentschrift: **04.05.94**

(51) Int. Cl.$^5$: **C07H 3/02**, C07H 9/02, C07H 13/04, C07H 15/04, A61K 31/70

(21) Anmeldenummer: **89123301.7**

(22) Anmeldetag: **15.12.89**

(54) **Neue Zuckerderivate, Verfahren zu ihrer Herstellung und ihre Verwendung.**

(43) Veröffentlichungstag der Anmeldung:
**19.06.91 Patentblatt 91/25**

(45) Bekanntmachung des Hinweises auf die Patenterteilung:
**04.05.94 Patentblatt 94/18**

(84) Benannte Vertragsstaaten:
**AT BE CH DE ES FR GB GR IT LI LU NL SE**

(56) Entgegenhaltungen:
**FR-A- 2 609 397**

**CHEMICAL ABSTRACTS, Band 64, Nr. 6, 14. März 1966, Spalte 8571, Zusammenfassung b, Columbus, Ohio, US; P.J. ANDERSON: "Oxidation of 3-deoxyxylitol by L-iditol dehydrogenase", & BIOCHIM. BIOPHYS. ACTA. 110(3), 627-9(1965)**

(73) Patentinhaber: **Laevosan-Gesellschaft m.b.H.
Estermannstrasse 17
A-4020 Linz(AT)**

(72) Erfinder: **Nitsch, Ernst,
Voltastrasse 33,
A-4040 Linz,(AT)**
Erfinder: **Lotz, Bernhard,
Roseggerstrasse 4,
A-4020 Linz,(AT)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)**

EP 0 432 309 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 64, Nr. 4, 14. Februar 1966, Spalte 5280, Zusammenfassung c, Columbus, Ohio, US; H. TSUCHIDA et al.: "Chemical reaction of sugars in aqueous alkaline solutions. I. Thin-layer chromatographic and paper-chromatographic identification of the reaction products in the reaction solution of glucose and lime water", & HYOGO NOKA DAIGAKU KENKYU HOKOKU, NOGEI-KAGAKU HEN 6(2), 73-80(1964)

JOURNAL OF WOOD CHEMISTRY AND TECHNOLOGY, Band 2, Nr. 2, 1982, Seiten 187-205, Marcel Dekker, Inc.; J.M. MacLEOD: "Alkaline degradation of cellobiose, 3,6-anhydro-4-o-(beta-D-glucopyranosyl)-D-glucose, 3,6-anhydro-4-o-methyl D-glucose, and D-glucose"

## Beschreibung

Die Erfindung betrifft neue Zuckerderivate, ein Verfahren zu ihrer Herstellung und Arzneimittel, die diese Verbindungen als Wirkstoff enthalten.

Unter Cytostatika werden chemotherapeutisch wirksame Stoffe verstanden, die eine hemmende Wirkung auf das Zellwachstum besitzen; da von dieser Hemmwirkung besonders schnell wachsende Zellen betroffen sind, wie sie in Tumoren und Leukämien vorkommen, versteht man unter Cytostatika in der Regel Chemotherapeutika gegen Krebs (Canzerostatica, Carzinostatica). Trotz umfangreicher Studien war es bisher nicht möglich, einen einheitlichen Wirkungsmechanismus für Cytostatika anzugeben. Die bisher bekannten und erprobten Cytostatika lassen sich etwa in die folgenden Gruppen einteilen: Alkylierende Verbindungen; cytostatische Naturstoffe; Antimetaboliten; Verbindungen unterschiedlicher Konstitution und Wirkungsweise. Ein schwerwiegender Nachteil der Cytostatika ist ihre hohe Toxizität, und darüber hinaus wirken viele Cytostatika selbst auch als Karzinogene und/oder Mutagene.

Aus Biochim. Biophys. Acta, 110 (1965) 627-629, ist bekannt, daß bei der Oxidation von 3-Deoxyxylitol durch L-Iditoldehydrogenase eine nicht eindeutig identifizierbare Substanz entsteht, von der vermutet wird, daß es sich um 3-Deoxyxylulose handelt. Eine pharmazeutische Anwendung dieser Substanz wird nicht vorgeschlagen.

Aufgabe der vorliegenden Erfindung war es deshalb, cytostatisch wirksame Verbindungen mit einer geringen Toxizität bereitzustellen.

Es wurde überraschenderweise gefunden, daß Bestimmte neue Zuckerderivate der nachstehend angegebenen allgemeinen Formel I und II eine hohe antiproliferative/cytotoxische Wirksamkeit bei einer sehr geringen Allgemeintoxizität aufweisen.

Gegenstand der Erfindung sind deshalb Arzneimittel, dadurch gekennzeichnet, daß sie als Wirkstoffe neue Zuckerderivate der allgemeinen Formeln I und/oder II

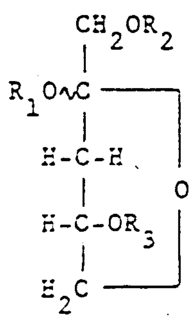

(I)

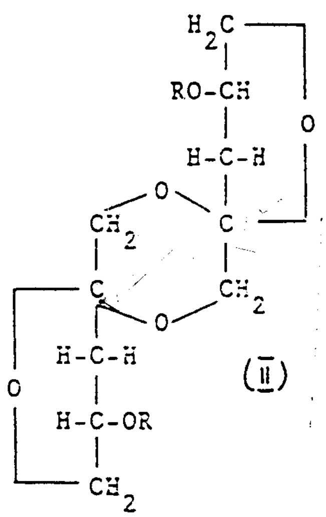

(II)

worin $R_1$ ein Wasserstoffatom oder eine Alkylgruppe oder Acylgruppe mit je 1 bis 7 Kohlenstoffatomen bedeutet, $R_2$ und $R_3$ ein Wasserstoffatom oder, wenn $R_1$ eine Alkylgruppe bedeutet, auch eine Acylgruppe $R_4CO$ bedeuten, R ein Wasserstoffatom oder eine Acylgruppe $R_5CO$ bedeutet und $R_4$ und $R_5$ eine Alkylgruppe mit 1 bis 7 Kohlenstoffatomen bedeuten, und übliche pharmazeutische Träger enthalten.

Eine Alkylgruppe $R_1$, $R_4$ oder $R_5$ kann verzweigt sein und ist vorzugsweise geradkettig; sie besitzt vorzugsweise 1 bis 4 Kohlenstoffatome und ist z.B. Methyl, Ethyl, Propyl oder n-Butyl. Eine Acylgruppe $R_1$ ist bevorzugt eine Acetylgruppe. $R_4$ und $R_5$ sind insbesondere Methyl. Die Reste $R_2$ und $R_3$ können gegebenenfalls auch verschieden sein, sind aber vorzugsweise gleich.

Bevorzugte Verbindungen der allgemeinen Formel I sind:

Methyl-3-Deoxy-D-Glyceropentulosid ($R_1$ = $CH_3$, $R_2$, $R_3$ = H), n-Butyl-3-Deoxy-D-Glyceropentulosid ($R_1$ = n-$C_4H_9$, $R_2$, $R_3$ = H), n-Butyl-Diacetyl-3-Deoxy-D-Glyceropentulosid ($R_1$ = n-$C_4H_9$, $R_2$, $R_3$ = $COCH_3$), und insbesondere 3-Deoxy-D-Glyceropentulose (3-DGP; $R_1$, $R_2$, $R_3$ = H); bevorzugte Verbindungen der allgemeinen Formel II sind Di-(3-Deoxy-D-Glyceropentulose)-Dianhydrid (R = H) und Diacetyl-di-3-(Deoxy-D-Glyceropentulose)-Dianhydrid (R = $COCH_3$).

Die erfindungsgemäße Verbindung der Formel I, worin $R_1$, $R_2$ und $R_3$ ein Wasserstoffatom bedeuten (3-Deoxy-D-Glyceropentulose; 3-DGP) wird durch alkalische Behandlung von alpha- oder beta-1,4-verknüpften Di- oder Oligosacchariden, die an ihrem reduzierenden Ende ein Glucose-, Fructose- oder Mannose-Molekül enthalten (Maltose, Maltulose, Epilactose, Lactose, Lactulose, Cellobiose, Oligomaltoside) gebildet. Sie entsteht bei der Behandlung dieser Di- und Oligosaccharide mit Alkalien, wie vorzugsweise z.B. mit Hydroxiden, Carbonaten oder Sulfiten der Alkali- oder Erdalkalimetalle oder mit organischen Basen, vorzugsweise z.B. Pyridin oder Triethylamin in einer Ausbeute von ca. 0,5 bis 5 %, bezogen auf eingesetztes Saccharid. Die 3-Deoxy-D-Glyceropentulose (3-DGP), ein bisher nicht bekannter Zucker, ist als 3-Deoxy-pentose isomer mit der 2-Deoxy-Ribose, welche in der Erbsubstanz eine wesentliche Rolle spielt.

Aus dem Gemisch der Reaktionsprodukte kann das 3-DGP auf verschiedene, an sich bekannte Weise angereichert bzw. rein gewonnen werden, z.B. durch Flüssig-Flüssig-Extraktion mit Ethanol, Aceton oder Acetonitril (diese ergeben mit konzentrierten Zuckerlösungen 2-phasige Gemische, in deren Oberphase 3-DGP stark angereichert ist), vorzugsweise aber durch Flüssig-Festchromatographie. Als stationäre Phasen besonders geeignet sind hierfür stark saure Kationenaustauscher aus vernetztem Polystyrol als Alkali- oder Erdalkalisalze, bevorzugt in der Na-, K- oder Ca-Form, sowie stark basische Anionenaustauscher aus vernetzem Polystyrol als Salze, bevorzugt in der Cl-, $SO_4$- oder $HSO_3$-Form. Bei Verwendung von z.B. Wasser als Eluent erscheinen im Eluat zuerst die unumgesetzten Zucker und ihre Isomerisierungsprodukte, dann gut abgetrennt das 3-DGP.

Aus den geeigneten Fraktionen kann hochgereinigte 3-DGP als farbloser, nicht kristallisierender Sirup erhalten werden. Da die Substanz unter wasserfreien oder wasserarmen Bedingungen zur Bildung von dimeren Anhydriden neigt, ist es zweckmäßig, sie als etwa 50 %ige wäßrige Lösung herzustellen und aufzubewahren. Auch die wäßrige Lösung ist aber relativ instabil und zersetzt sich langsam unter Braunfärbung, Trübung und Bildung verschiedener Furankörper. Erhöhung der Temperatur und Absenken des pH-Wertes beschleunigen diese Zersetzung. Für die Aufbewahrung ist es daher zweckmäßig, Temperaturen von $\leqq 4°C$ und pH-Werte zwischen 5 und 6 einzuhalten. In solchen Lösungen liegt offenbar ein Gemisch der alpha- und beta-Furanosen und des offenkettigen Ketozuckers vor.

3-DGP ist in dieser Form biologisch aktiv. Anstelle des freien 3-DGP können aber auch stabilere Derivate eingesetzt werden, nämlich die entsprechenden Glycoside oder Diacylverbindungen (Verbindungen der Formel I, worin $R_1$ einen Alkylrest bedeutet und $R_2$ und $R_3$ ein Wasserstoffatom oder einen Acylrest $R_4CO$), in der Regel nicht kristallisierende, ölige Verbindungen, oder das Dianhydrid der Formel II ($R = H$) oder dessen Diacylester der Formel II ($R$ = Acyl), bei denen es sich in der Regel um gut kristallisierende Verbindungen handelt.

Die Glycoside bilden sich außerordentlich leicht aus den Lösungen von 3-DGP in den entsprechenden Alkoholen in Gegenwart sehr geringer Mengen an Säure oder Ionenaustauscher in der $H^+$-Form als Katalysator bereits nach kurzer Behandlung bei Raumtemperatur. Ebenso leicht tritt in wäßriger Lösung Hydrolyse der Glycoside ein, so daß unter physiologischen Bedingungen eine Äquivalenz mit freier 3-DGP erwartet werden kann.

Das Dianhydrid der Formel II ($R = H$) bildet sich leicht bei Behandlung von mäßig sauren 3-DGP-Lösungen mit wasserentziehenden Mitteln, wie z.B. wasserfreiem Natriumsulfat oder Calciumsulfat, oder beim Eindampfen von wäßrigen oder organischen Lösungen von 3-DGP und Stehenlassen des wasserfreien Produktes. Das Dianhydrid (Formel II) ist ähnlich den Glycosiden in wäßriger Lösung leicht zum Monomeren spaltbar.

Die Acylverbindungen der Formel I und II, worin $R_2$ und $R_3$ bzw. R eine Acylgruppe bedeuten, lassen sich aus den Verbindungen der Formel I, worin $R_1$ eine Alkylgruppe (Glycoside) oder Acylgruppe ist, oder dem Dianhydrid der Formel II, worin R ein Wasser-stoffatom ist, auf an sich bekannte Weise, z.B. durch Umsetzung mit Essigsäureanhydrid in Pyridin, herstellen.

Gegenstand der Erfindung ist deshalb auch ein Verfahren zur Herstellung der erfindungsgemäßen Verbindungen der allgemeinen Formel I oder II, worin $R_1$, $R_2$, $R_3$, $R_4$, $R_5$ und R die vorstehend genannte Bedeutung besitzen, das dadurch gekennzeichnet ist, daß man alpha- oder beta-1,4-verknüpfte Di- oder Oligosaccharide, die an ihrem reduzierenden Ende ein Glucose-, Fructose- oder Mannose-Molekül enthalten, mit anorganischen oder organischen Basen behandelt, aus dem erhaltenen Reaktionsgemisch die 3-Deoxy-D-Glyceropentulose (3-DGP) der Formel I, worin $R_1$, $R_2$ und $R_3$ ein Wasserstoffatom bedeuten, auf an sich bekannte Weise isoliert, und, wenn erwünscht, das 3-DGP auf an sich bekannte Weise in sein Glycosid (Formel I, $R_1$ = Alkyl), Diacylglycosid (Formel I, $R_1$ = Alkyl, $R_2$, $R_3$ = $R_4CO$), Dianhydrid (Formel II, $R = H$) oder Diacyldianhydrid (Formel II, $R = R_5CO$) überführt.

Vorzugsweise wird das Verfahren im wäßrigen Medium durchgeführt. Die Temperatur der Umsetzung liegt vorzugsweise zwischen Raumtemperatur und 100 °C.

Zur Prüfung auf ihre antiproliferativen/cytotoxischen Eigenschaften wurden die erfindungsgemäßen Verbindungen der Formel I und II mittels einer humanen Ovarialkarzinom-Zellkultur (HOC-7) getestet, wobei als Testsystem der sogenannte human tumour cloning assay (HTCA) herangezogen wurde, ein System, in dem das klonale Wachstum maligner Einzelzellen in semisoliden Medien selektiv erfaßt wird (vgl. S. Hamburger, Science 197, (1977), 461). Dabei zeigten die Verbindungen eine überraschend hohe antiproliferative/cytotoxische Wirkung.

Bei der Toxizitätsprüfung (Maus, oral) wurde gefunden, daß die erfindungsgemäßen Verbindungen der Formel I und II keine erkennbare akute Toxizität aufweisen.

Aufgrund ihrer bemerkenswerten antiproliferativen/cytotoxischen Wirksamkeit und des günstigen Toxizitätsbefundes weisen die erfindungsgemäßen Verbindungen einen ungewöhnlich breiten pharmakologischen Index (Koeffizient aus effektiver Dosis ED und Toxizität $LD_{50}$) auf. Außerdem wurde eine bakterizide Wirksamkeit festgestellt.

Gegenstand der Erfindung sind deshalb auch Arzneimittel, die eine oder mehrere der Verbindungen der Formel I und/oder II als Wirkstoff enthalten. Neben den üblichen pharmazeutischen Konfektionierungs-(Träger)- und/oder Verdünnungsmitteln können diese Arzneimittel neben den Verbindungen der Formel I und/oder II zur Unterstützung der Therapie gegebenenfalls auch noch weitere Wirkstoffe enthalten, sofern diese mit den erfindungsgemäßen Verbindungen zusammen keine unerwünschten Nebenwirkungen zeigen. Die geeigneten, zweckmäßigen Verabreichungsformen, Dosierungs- und Anwendungsweisen für z.B. orale, topische, infundierende, injizierende usw. Applikation, entsprechen im wesentlichen den für anerkannte antiproliferativ/cytotoxisch wirkende Mittel bekannten Bedingungen, wobei aber aufgrund der besseren therapeutischen Breite (wesentlich geringere Toxizität) der erfindungsgemäßen Verbindungen höhere Dosierungen und/oder eine häufigere Verabreichung in Frage kommen.
Sie kann ein- oder mehrmals am Tag verabreicht werden. Die zweckmäßigste Menge und Häufigkeit der Verabreichung richtet sich dabei insbesondere nach der Art und Schwere der Erkrankung und nach dem allgemeinen Befinden des Patienten.

In den nachstenden Tabellen 1 bis 3 werden die Rf-Werte erfindungsgemäßer Verbindungen, die zu ihrer Ermittlung verwendeten DC-Fließmittel und HPLC-Systeme angegeben.

Tabelle 1

| Dünnschicht-Chromatographie (DC) | | |
|---|---|---|
| | Fließmittel$^{+++}$ | $R_F$-Werte |
| 3-DGP | 1 | 0,49 |
| 3-DGP | 2 | 0,29 |
| 3-DGP | 4 | 0,17$^{+}$ |
| Methyl-DGP | 4 | 0,42 u. 0,55$^{+}$ |
| Ethyl-DGP | 4 | 0,67 |
| i-Propyl-DGP | 4 | 0,90 |
| n-Butyl-DGP | 4 | 1,0$^{+}$, Laufstr. 76 mm |
| n-Butyl-DGP-Diacetat | 3 | 0,73 u. 0,64$^{++}$ |
| Di-DGP-Dianhydrid | 2 | 0,35 |
| Di-DGP-Dianyhdrid | 4 | 0,25$^{+}$ |
| DGP-4-Nitrophenylosazon | 5 | 0,21 |
| Di-DGP-Dianhydrid-diacetat | 4 | 0,64 |
| 3-DGP-triacetat | 4 | 0,74 |

$^{+}$ $R_r$-Werte für n-Butyl-DGP = 1,0; Laufstrecke 3 x 10 cm
$^{++}$ Laufstrecke 2 x 10 cm
$^{+++}$siehe Tabelle 2

Es wurden folgende DC-Fließmittel und HPLC-Systeme verwendet:

Tabelle 2

DC Fließmittel

| Nr. | (DC-Fertigplatten Kieselgel 60 F254 10x20 cm MERCK Best.Nr. 5729) |
|---|---|
| 1 | Methylethylketon/Eisessig/Methanol<br>60 20 20 |
| 2 | Methylethylketon/Wasser<br>92,5 7,5 |
| 3 | Methylethylketon/Toluol<br>20 80 |
| 4 | Methylethylketon/Toluol<br>50 50 |
| 5 | Benzol/Tetrahydrofuran/Eisessig<br>75 20 5 |

Sprühreagenz: 200 mg 1,3-Naphthalindiol in 50 ml Methanol + 50 ml Schwefelsäure 20 %; 10 Minuten 100°C

3-DGP und Derivate erscheinen als blaugrüne Flecken.

Tabelle 3

| HPLC-Systeme | | |
|---|---|---|
| | System 1 | System 2 |
| Säule | SHODEX S 801 8 x 500 mm | Vertex-Hypersil APS 3 µm 4,0 x 250 mm |
| Eluent | Calciumazid in $H_2O$ 0,02 % pH 5,1 | Acetonitril-Phosphatpuffer 0,01M pH 7,0 80 + 20 |
| Fluß ml/Minute | 0,9 | 1,2 |
| Detektor | RI | RI |
| Aufgabevolumen µl | 20 | 20 |
| Temperatur °C | 60 | Raumtemperatur |
| Retentionszeiten ca. Minuten: | | |
| 3-DGP | 26,80 (Fig.4) | 3,29 (Fig.5) |
| Dianhydrid (II, R = H) | 25,54 | -- |

Die nachfolgenden Beispiele sollen die Erfindung näher erläutern.

**Beispiel 1**

Herstellung von 3-DGP aus Lactose

52,63 g Lactose-Monohydrat, entsprechend 50,0 g wasserfreier Lactose, wurden in 25 g Wasser bei 100°C gelöst, 0,65 g wasserfreies Natriumsulfit zugesetzt, 20 Minuten bei 100°C gehalten, rasch auf Raumtemperatur abgekühlt, mit ca. 1 mg Lactose-Monohydrat angeimpft und nach ca. 24 Stunden die Mutterlauge unter Nachwaschen mit wenig eiskaltem Wasser von den Kristallen der unumgesetzten Lactose abgesaugt.

Das Filtrat enthielt in 36,4 g Lösung 9,5 g Saccharide. Es wurde über Mischbettaustauscher entsalzt und die erhaltene neutrale Lösung auf 20 g im Vakuum eingedampft.

Das Produkt wurde auf eine Glassäule (innerer Durchmesser 26 mm, mit Mantelheizung auf 60°C, Länge 100 cm, gefüllt mit ca. 500 ml Kationenaustauscher LEWATIT MDS 1368 (stark sauer, Polystyrol-Matrix, Hersteller BAYER AG, Leverkusen, in der Natriumform)) aufgegeben und mit Wasser eluiert. Nach einem substanzfreien Vorlauf von 175 ml wurde eine Fraktion von 110 ml aufgefangen, welche die Hauptmenge Lactose und Begleitsaccharide enthielt. Eine weitere Fraktion von 150 ml enthielt das gesamte 3-DGP mit geringen Mengen von Begleitsacchariden.

Die 3-DGP-Fraktionen aus insgesamt 10 Reaktionsansätzen wurden gesammelt, im Vakuum auf ca. 20 g eingedampft und erneut über das gleiche System chromatographiert. Die 3-DGP-Fraktion enthielt nurmehr Spuren von Begleitzuckern. Sie wurde im Vakuum auf 10 g eingedampft.

| | |
|---|---|
| Wassergehalt nach Karl Fischer: | 49,2 % |
| HPLC-Analyse (System 1) ergab: | 48,9 % 3-DGP<br>0,8 % Galactose<br>1,1 % andere Zucker |
| Spezifische Drehung: | -22,1° (c = 3,0 in Wasser) |

Dieses Produkt wurde für die in Beispiel 3 beschriebenen Versuche an Zellkulturen eingesetzt.

Strukturaufklärung:

Die neue Substanz reduziert Fehlingsche Lösung, nicht aber Brom in schwach saurem Milieu. Es handelt sich also um eine Ketose. Sie gibt die Reaktionen nach Keller-Kiliani (H. Kiliani, Ar. 234, (1896), 273) und nach Dische (Z. Dische, Mikroch. 8, (1930), 4-32) auf Deoxyzucker. Spezifische Drehung [alpha]-

$^{20}{}_D$ = -22,1° (c = 3,0 in Wasser). Sie verbraucht pro Mol 1 Mol Perjodat, besitzt also nur eine Diolgruppierung. Das kristallisierte Nitrophenylosazon erwies sich, wie zu erwarten, im Schmelzpunkt (253°C) und in der Elementaranalyse als identisch mit dem für 3-Deoxy-Xylose beschriebenen Produkt (S. Mukherjee und A.R. Todd, Soc. 94 (1947), 969-973). $R_F$-Wert in Fließmittel 5:0,21. Da das Methylglycosid (I, $R_1$ = $CH_3$, $R_2$, $R_3$ = H), das n-Butyl(1)-Glycosid (I, $R_1$ = n-$C_4H_9$, $R_2$, $R_3$ = H) und das Dianhydrid (II, R = H) kein Perjodat verbrauchen, ist eine Hydroxylgruppe der glycosidischen Gruppe benachbart. Das n-Butyl-Glycosid (I, $R_1$ = n-$C_4H_9$, $R_2$, $R_3$ = H) läßt sich in Pyridin mit Essigsäureanhydrid zu einer Diacetylverbindung (I, $R_1$ = n-$C_4H_9$, $R_2$, $R_3$ = $COCH_3$) umsetzen, welche im Hochvakuum destilliert werden kann. $K_p$: 90°C (0,002 Torr = 0,26 Pa). Verseifung mit NaOH ergab ein Molekulargewicht von 272,5 (berechnet für $C_{13}H_{22}O_6$: 274,319). Das IR-Spektrum (Figur 1) und das Massen- und NMR-Spektrum (Figur 2) bestätigen die angegebene Struktur.

| Elementaranalyse ($C_{13}H_{22}O_6$) | | | |
|---|---|---|---|
| gefunden: | 56,61 % C, | 7,93 % H, | 35,6 % O |
| berechnet: | 57,0 % C, | 8,09 % H, | 35,0 % O |

HPLC siehe Tab. 3

DC siehe Tab. 1

**Beispiel 2**

Herstellung von Di-(3-deoxy-D-glyceropentulose)-dianhydrid (II, R = H)

5 g des in Beispiel 1 erhaltenen Produktes, enthaltend 2,445 g 3-DGP wurden im Wasserstrahlvakuum bei ca. 50°C Badtemperatur weitgehendst eingedampft, der verbleibende Sirup in 10 ml Methylethylketon aufgenommen, erneut eingedampft und derselbe Vorgang noch viermal wiederholt. Zuletzt wurde der Trockenrückstand noch 1 Stunde im Vakuum auf 100°C erhitzt. Nach mehrwöchigem Stehen im Vakuum trat spontane Kristallisation zu einem dicken Kristallbrei ein. Durch Umkristallisation aus 1-Butanol und Methanol wurden 0,8 g farblose Kristalle erhalten. Schmelzpunkt: 148-149°C.

Spezifische Drehung/alpha/$^{20}{}_D$ = -12,0°C (c = 2,3 in Wasser)

| Elementaranalyse: | | | |
|---|---|---|---|
| gefunden: | 51,35 % C, | 6,83 % H, | 41,26 % O |
| berechnet: | 51,72 % C, | 6,94 % H, | 41,34 % O (für $C_{10}H_{16}O_6$) |

Das IR-Spektrum zeigt Figur 3.

Die Substanz reduziert Fehlingsche Lösung nicht und verbraucht in neutraler Lösung kein Perjodat.

Die mit Essigsäureanhydrid/Pyridin erhältliche Diacetylverbindung (II, R = Acetyl) ist ebenfalls eine gut kritallisierende Substanz.

DC siehe Tabelle 1.

**Beispiel 3:**

DGP-Ausbeute aus Lactose mit verschiedenen Reaktanden

Jeweils 52,63 g Lactose-Monohydrat, entsprechend 50 g wasserfreier Lactose wurden wie in Beispiel 1 in 25 g Wasser bei 100°C gelöst, 25 ml der Lösung des Reaktanden (Konzentration lt. Tabelle 1) zugegeben und 20 Minuten bei 100°C gehalten. Nach raschem Abkühlen wurde mit HPLC-System 1 der DGP-Gehalt des Reaktionsgemisches in %, bezogen auf eingesetzte wasserfreie Lactose, berechnet. Ergebnisse erscheinen in der folgenden Tabelle 4.

Tabelle 4

| Reaktand | Konzentration g/100 ml | % DGP gebildet |
|---|---|---|
| $Na_2SO_3$ | 5 | 0,96 |
| $Na_2CO_3$ | 4 | 0,17 |
| NaOH | 1,5 | 2,01 |
| Triethylamin | 0,25 ml + 0,25 ml $H_2O$ | 0,34 |
| Pyridin | 0,25 ml | 0,13 |

**Beispiel 4**

DGP-Ausbeute aus verschiedenen Di- und Oligosacchariden

Durchführung wie in Beispiel 3. Statt Lactose wurden jeweils 50 g (Trockenmasse) verschiedener Di- und Oligosaccharide eingesetzt. Als Reaktand wurden je 25 ml einer Natriumhydroxidlösung 1,5 g/100 ml zugegeben.
Die Ergebnisse sind in der folgenden Tabelle 5 dargestellt.

Tabelle 5

| Saccharid eingesetzt | % DGP gebildet |
|---|---|
| Lactulose | 2,07 |
| Maltose | 1,54 |
| Epilactose | 1,34 |
| Oligomaltoside+ | 1,51 |
| Cellobiose | 1,75 |

+ Glucosefrei, aus Stärkesirup durch Umfällen mit Methanol Mn ca. 2300

**Beispiel 5**

Herstellung von n-Butyl-3-Deoxy-D-Glyceropentulosid

5 g des in Beispiel 1 erhaltenen Produktes, enthaltend 2,445 g DGP wurden mit 5 ml n-Butanol versetzt und im Vakuum-Rotationsverdampfer etwa auf Ausgangsvolumen eingedampft. Der Vorgang wurde noch dreimal zur Beseitigung allen Wassers wiederholt, das Volumen mit n-Butanol auf 25 ml ergänzt und bei Raumtemperatur 10 Minuten nach Zusatz von 0,5 g fein gepulvertem stark sauren Kationenaustauscher in der $H^+$-Form (DUOLITE Microionex H) gerührt. Nach Filtration und Zusatz von 50 µl Pyridin wurde im Vakuum alles Lösungsmittel abdestilliert. Es verblieben 3,40 g einer hellgelben, öligen Flüssigkeit, gut löslich in Wasser, Alkoholen und niederen Ketonen. Dünnschicht-Chromatographie s. Tabelle 1.

**Beispiel 6**

Herstellung von n-Butyl-1,4-Diacetyl-3-Deoxy-D-Glyceropentulosid

3,40 g des nach Beispiel 4 hergestellten n-Butyl-Glycosides wurden in 100 ml Pyridin gelöst, 10 ml Essigsäureanhydrid zugesetzt und die Mischung 24 h bei Raumtemperatur stehengelassen. Der Ansatz wurde sodann in 1 l Eiswasser unter Rühren eingegossen, das ausgefallene farblose Öl mehrmals mit Wasser nachgewaschen und im Vakuum über $P_2O_5$ getrocknet.
Ausbeute: 3,67 g.
Das Produkt ließ sich im Hochvakuum von 0,002 mm Hg bei ca. 80 °C destillieren.
Spezifische Drehung/alpha$^{20}/_{D}$ = -37,7 ° (c = 0,5; Chloroform)
Dichte 20/4: 1,86

| Elementaranalyse: | | | |
|---|---|---|---|
| | C | H | O |
| für $C_{13}H_{22}O_6$ berechnet: | 56,61 | 7,93 | 35,6 |
| gefunden: | 57,0 | 8,09 | 35,0 |

IR-Spektrum siehe Fig. 1
HNMR-Spektrum siehe Fig. 2
Dünnschicht-Chromatographie siehe Tabelle 1

**Beispiel 7**

Herstellung von 3-DGP-Triacetat ($R_1$ : Acyl)

3,16 g Di-DGP-Diacetat wurden in 10 ml 0,1n HCl in Eisessig gelöst, 0,5 ml Essigsäureanhydrid zugegeben und 120 Minuten bei 40 °C gehalten. Nach Zusatz von 200 µl Triethylamin wurde der Ansatz im Vakuum eingedampft. Es hinterblieb ein gelbes Öl, 5,15 g. Es wurde in 5 ml Toluol gelöst, auf eine Säule mit 200 ml Kieselgel 60 MERCK (230 - 400 mesh) aufgegeben, zunächst mit 200 ml Toluol, dann mit 200 ml Toluol-Ethylacetat 80 + 20 und mit 200 ml Toluol-Ethylacetat 60 + 40 eluiert. In der Fraktion 310-360 ml erschien die Hauptsubstanz. Sie wurde im Vakuum eingedampft. Farbloses Öl. Ausbeute: 3,62 g.

$R_F$-Wert siehe Tabelle 1.

**Beispiel 8**

Biologische Testung

Das in Beispiel 1 hergestellte 3-DGP sowie DGP-DA von Beispiel 2 wurden hinsichtlich ihrer antiproliferativen/cytotoxischen Eigenschaften mittels einer humanen Ovarialkarzinom-Zellkultur (HOC-7) getestet, wobei als Testsystem der sogenannte human tumour cloning assay (HTCA) herangezogen wurde, ein System, in dem das klonale Wachstum maligner Einzelzellen in semisoliden Medien selektiv erfaßt wird (vgl. S. Hamburger, Science 197, (1977), 461).

Methode

Je eine wäßrige Lösung von 3-DGP und DGP-DA wurde in folgender Verdünnungsreihe getestet.

| Testkonzentration | Verdünnung im Röhrchen | Verdünnung im Petrischälchen | % | µ g/ml |
|---|---|---|---|---|
| | | | im Petrischälchen | |
| C | 1:5 | 1:10 | 0,5 | 5.000 |
| D | 1:10 | 1:20 | 0,25 | 2.500 |
| E | 1:50 | 1:100 | 0,05 | 500 |
| F | 1:100 | 1:200 | 0,025 | 250 |
| F1 | 1:125 | 1:250 | 0,02 | 200 |
| F2 | 1:165 | 1:330 | 0,015 | 152 |
| F3 | 1:250 | 1:500 | 0,01 | 100 |
| G | 1:500 | 1:1.000 | 0,005 | 50 |
| H | 1:1.000 | 1:2.000 | 0,0025 | 25 |
| I | 1:5.000 | 1:10.000 | 0,0005 | 5 |
| J | 1:10.000 | 1:20.000 | 0,00025 | 2,5 |

wobei sowohl ein Negativkontrollansatz (in Form von $HgCl_2$) als auch ein Ansatz mit Lösungsmittel (Aqua bidest) mitgeführt wurde.

Die Kulturen wurden bei 37 °C, 5 % $CO_2$, 100 % Luftfeuchtigkeit 21 Tage inkubiert, danach wurden die Kultursysteme aufgearbeitet, wobei die gewachsenen Kolonien (Klone) unter dem Mikroskop ausgezählt wurden.

Als Wirksamkeit der getesteten Lösung wurde ein Kolonienüberleben < 50 % der Kontrolle definiert.

Ergebnis

Lösungsmittelkontrolle: VII: Kolonienüberleben 92 %
= keine Hemmung des Kolonienwachstums
Negativkontrolle: VI: Kolonienüberleben 0 %
Linearitätskontrolle: r = 0,968
Testsubstanzkonzentration: DGP

| | | |
|---|---|---|
| C-F: | Kolonienüberleben | 0 % = Wirksamkeit |
| F1: | | 0,08 % = Wirksamkeit |
| F2: | | 10 % = Wirksamkeit |
| F3: | | 44 % = intermediäre Wirksamkeit |
| G-J: | | 65 - 84 % = keine Wirksamkeit |

Testsubstanzkonzentration: DGP-DA

| | | |
|---|---|---|
| C: | Kolonienüberleben | 30 % = Wirksamkeit |
| D: | | 50 % = intermediäre Wirksamkeit |
| E: | | 57 % = intermediäre Wirksamkeit |
| F-J: | | 61 - 89 % = keine Wirksamkeit |

Das Ergebnis zeigt eine scharfe Trennlinie zwischen Sensitivität und Resistenz der getesteten Ovarialkarzinomlinie, wobei im sensitiven Bereich eine hohe antiproliferative/cytotoxische Wirksamkeit nachgewiesen werden konnte.

Mit den in Beispiel 1 beschriebenen Derivaten von 3-DGP (Methylglycosid, n-Butylglycosid, Diacyl-n-butyl-glycosid) wurde unter gleichen Testbedingungen ebenfalls eine eindeutige antiproliferative/cytotoxische Wirkung erhalten.

**Beispiel 9**

Konservierung nach DAB 9, VIII, N1

Die erfindungsgemäßen Verbindungen weisen eine bakterizide Wirksamkeit auf, wie die nachstehend angegebenen Versuchsergebnisse zeigen:

Präparat: Di-deoxy-glyceropentulose-Dianhydrid 0,3 %, 1 % und 3 %

Die Untersuchung wurde mit folgenden Keimen durchgeführt:
Escherichia coli ATCC 8739
Pseudomonas aeruginosa ATCC 9027
Staphylococcus aureus ATCC 6538
Candida albicans ATCC 10231
Aspergillus niger ATCC 16404

Tabelle 6

| Testorganismen | Konz | KBE/ml zu Beginn d. Prüfung | KBE nach 1 Tag | KBE nach 3 Tagen | KBE nach 10 Tagen |
|---|---|---|---|---|---|
| E. coli | 0,3 % | $1,43 \times 10^5$ | $1,0 \times 10^1$ | 0 | 0 |
| Pseud.aerug. | | $1,47 \times 10^5$ | $4,30 \times 10^3$ | $3,39 \times 10^4$ | $1,10 \times 10^6$ |
| Staph. aureus | | $3,74 \times 10^5$ | 0 | 0 | 0 |
| Cand. albicans | | $3,40 \times 10^5$ | $1,14 \times 10^6$ | $1,61 \times 10^6$ | $1,92 \times 10^6$ |
| Asp. niger | | $3,61 \times 10^5$ | $3,05 \times 10^5$ | $5,85 \times 10^5$ | $4,15 \times 10^5$ |
| E. coli | 1 % | $0,70 \times 10^5$ | 0 | 0 | 0 |
| Pseud. aerug. | | $1,96 \times 10^5$ | $4,20 \times 10^2$ | $4,23 \times 10^3$ | $5,88 \times 10^5$ |
| Staph. aureus | | $4,25 \times 10^5$ | 0 | 0 | 0 |
| Cand. albicans | | $3,25 \times 10^5$ | $4,94 \times 10^5$ | $1,60 \times 10^6$ | $1,95 \times 10^6$ |
| Asp. niger | | $2,87 \times 10^5$ | $3,90 \times 10^5$ | $3,30 \times 10^5$ | $5,30 \times 10^5$ |
| E. coli | 3 % | $0,65 \times 10^5$ | 0 | 0 | 0 |
| Pseud. aerug. | | $1,80 \times 10^5$ | 0 | 0 | 0 |
| Staph. aureus | | $3,70 \times 10^5$ | 0 | 0 | 0 |
| Cand. albicans | | $3,19 \times 10^5$ | $5,65 \times 10^5$ | $1,80 \times 10^6$ | $2,12 \times 10^6$ |
| Asp. niger | | $2,19 \times 10^5$ | $4,27 \times 10^5$ | $3,50 \times 10^5$ | $4,55 \times 10^5$ |

**Beispiel 10**

Konservierung nach DAB 9, VIII N1

Analog Beispiel 9 wurde die bakterizide Wirksamkeit von DGP untersucht.

Präparat: DGP-wässriger Sirup 0,3 %, 1 % und 3 %,

Charge: 91621

Die Untersuchung wurde mit folgenden Keimen durchgeführt:

Escherichia coli ATCC 8739

Pseudomonas aeruginosa ATCC 9027

Staphyloccus aureus ATCC 6538

Candida albicans ATCC 10231

Aspergillus niger ATCC 16404

Tabelle 7

| Testorganismen | Konz. | KBE/ml zu Beginn der Prüfung | KBE nach 1 Tag | KBE nach 3 Tagen | KBE nach 10 Tagen |
|---|---|---|---|---|---|
| E. coli | | $1,1x10^6$ | $6,7x10^5$ | $3,9x10^5$ | $1,7x10^6$ |
| Pseud. aerug. | | $2,2x10^5$ | $1,9x10^5$ | $8,4x10^3$ | 0 |
| Staph. aureus | 0,3 % | $1,8x10^5$ | $2,8x10^5$ | $1,5x10^5$ | 0 |
| Cand. albicans | | $9,1x10^5$ | $4,8x10^6$ | $4,9x10^6$ | $3,3x10^6$ |
| Asp. niger | | $1,9x10^4$ | $5,5x10^3$ | $1,3x10^4$ | $2,4x10^4$ |
| E. coli | | $1,2x10^6$ | $5,9x10^5$ | $5,4x10^5$ | $<10^2$ |
| Pseud. aerug. | | $2,2x10^5$ | $7,2x10^4$ | 0 | 0 |
| Staph. aureus | 1 % | $1,7x10^5$ | $1,3x10^5$ | $3x10^4$ | 0 |
| Cand. albicans | | $1,3x10^6$ | $4,3x10^6$ | $9x10^6$ | $3,9x10^6$ |
| Asp. niger | | $1,7x10^4$ | $6x10^3$ | $1,3x10^4$ | $1,0x10^4$ |
| E. coli | | $1,2x10^6$ | $4,4x10^5$ | $2,8x10^3$ | 0 |
| Pseud. aeruginosa | | $2,2x10^5$ | 15 | 0 | 0 |
| Staph. aureus | 3 % | $1,4x10^5$ | $4,5x10^4$ | 0 | 0 |
| Cand. albicans | | $1,1x10^6$ | $3,8x10^6$ | $1,1x10^7$ | $4,0x10^6$ |
| Asp. niger | | $1,1x10^4$ | $4x10^3$ | $4,5x10^4$ | $3,6x10^4$ |

**Patentansprüche**

**Patentansprüche für folgende Vertragsstaaten : DE, GB, FR, IT, NL, SE, LI, CH, BE, AT, LU**

**1.** Arzneimittel, dadurch gekennzeichnet, daß es als Wirkstoff eine oder mehrere der Verbindungen der allgemeinen Formel I und/oder II

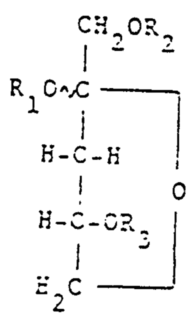

(I)

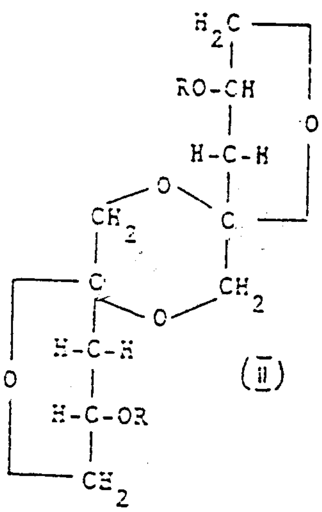

(II)

worin $R_1$ ein Wasserstoffatom oder eine Alkylgruppe oder Acylgruppe mit je 1 bis 7 Kohlenstoffatomen bedeutet, $R_2$ und $R_3$ ein Wasserstoffatom oder, wenn $R_1$ eine Alkylgruppe ist, auch eine Acylgruppe $R_4CO$ bedeuten, R ein Wasserstoffatom oder eine Acylgruppe $R_5CO$ bedeutet, und $R_4$ und $R_5$ eine Alkylgruppe mit 1 bis 7 Kohlenstoffatomen bedeuten, und übliche pharmazeutische Träger- und/oder Verdünnungsmittel enthält.

**2.** Verfahren zur Herstellung der Wirkstoffe der Formel I und II nach Anspruch 1, worin $R_1$, $R_2$, $R_3$, $R_4$, $R_5$ und R die in Anspruch 1 angegebenen Bedeutungen besitzen,
**dadurch gekennzeichnet,**
daß man $\alpha$- oder $\beta$-1,4-verknüpfte Di- oder Oligosaccharide, die an ihrem reduzierenden Ende ein

Glucose-, Fructose-oder Mannose-Molekül enthalten, mit anorganischen oder organischen Basen behandelt, aus der erhaltenen Reaktionsmischung die 3-Deoxy-D-Glyceropentulose (3-DGP) der Formel I, worin $R_1$, $R_2$ und $R_3$ ein Wasserstoffatom bedeuten, isoliert, und, wenn erwünscht, das 3-DGP auf an sich bekannte Weise in sein Glycosid (Formel I, $R_1$ = Alkyl), Diacylglycosid (Formel I, $R_1$ = Alkyl, $R_2$, $R_3$ = $R_4CO$), Dianhydrid (Formel II, R = H) oder Diacyldianhydrid (Formel II, R = $R_5CO$) überführt.

**3.** Methyl-3-Deoxy-D-Glyceropentulosid (Formel I, $R_1$ = $CH_3$, $R_2$, $R_3$ = H).

**4.** n-Butyl-3-Deoxy-D-Glyceropentulosid (Formel I, $R_1$ = n-$C_4H_9$, $R_2$, $R_3$ = H).

**5.** n-Butyl-Diacetyl-3-Deoxy-D-Glyceropentulosid (Formel I, $R_1$ = n-$C_4H_9$, $R_2$, $R_3$ = $COCH_3$).

**6.** Di-(3-Deoxy-D-Glyceropentulose)-Dianhydrid (Formel II, R = H).

**7.** Diacetyl-Di-(3-Deoxy-D-Glyceropentulose)-Dianhydrid (Formel II, R = $COCH_3$).

**8.** Verwendung einer oder mehrerer Verbindungen der allgemeinen Formel I und/oder II zur Herstellung eines anti-proliferativen/cytotoxischen Arzneimittels.

**Patentansprüche für folgenden Vertragsstaat : ES**

**1.** Verwendung einer oder mehrerer Verbindungen der allgemeinen Formel I und/oder II

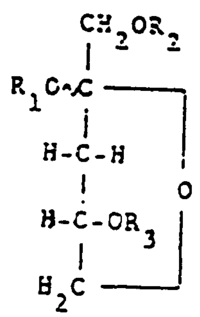

(I)

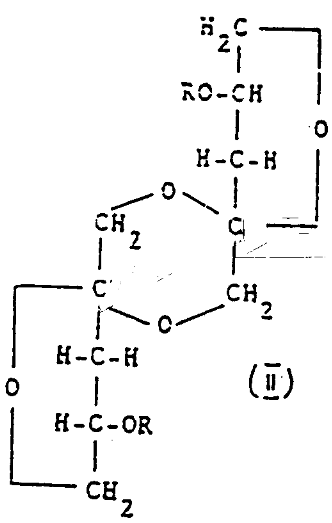

(II)

worin $R_1$ ein Wasserstoffatom oder eine Alkylgruppe oder Acylgruppe mit je 1 bis 7 Kohlenstoffatomen bedeutet, $R_2$ und $R_3$ ein Wasserstoffatom oder, wenn $R_1$ eine Alkylgruppe ist, auch eine Acylgruppe $R_4CO$ bedeuten, R ein Wasserstoffatom oder eine Acylgruppe $R_5CO$ bedeutet, und $R_4$ und $R_5$ eine Alkylgruppe mit 1 bis 7 Kohlenstoffatomen bedeuten, und übliche pharmazeutische Träger- und/oder Verdünnungsmittel enthält
zur Herstellung eines anti-proliferativen cytotoxischen Arzneimittels.

**2.** Verfahren zur Herstellung der Wirkstoffe der Formel I und II nach Anspruch 1, worin $R_1$, $R_2$, $R_3$, $R_4$, $R_5$ und R die in Anspruch 1 angegebenen Bedeutungen besitzen,
**dadurch gekennzeichnet,**
daß man $\alpha$- oder $\beta$-1,4-verknüpfte Di- oder Oligosaccharide, die an ihrem reduzierenden Ende ein Glucose-, Fructose- oder Mannose-Molekül enthalten, mit anorganischen oder organischen Basen behandelt, aus der erhaltenen Reaktionsmischung die 3-Deoxy-D-Glyceropentulose (3-DGP) der Formel I, worin $R_1$, $R_2$ und $R_3$ ein Wasserstoffatom bedeuten, isoliert, und, wenn erwünscht, das 3-DGP auf an sich bekannte Weise in sein Glycosid (Formel I, $R_1$ = Alkyl), Diacylglycosid (Formel I, $R_1$ = Alkyl, $R_2$, $R_3$ = $R_4CO$), Dianhydrid (Formel II, R = H) oder Diacyldianhydrid (Formel II, R = $R_5CO$) überführt.

3. Verfahren nach Anspruch 2, dadurch gekennzeichnet, daß man Methyl-3-Deoxy-D-Glyceropentulosid (Formel I, $R_1 = CH_3$, $R_2$, $R_3 = H$) herstellt.

4. Verfahren nach Anspruch 2, dadurch gekennzeichnet, daß man n-Butyl-3-Deoxy-D-Glyceropentulosid (Formel I, $R_1 = n\text{-}C_4H_9$, $R_2$, $R_3 = H$) herstellt.

5. Verfahren nach Anspruch 2, dadurch gekennzeichnet, daß man n-Butyl-Diacetyl-3-Deoxy-D-Glyceropentulosid (Formel I, $R_1 = n\text{-}C_4H_9$, $R_2$, $R_3 = COCH_3$) herstellt.

6. Verfahren nach Anspruch 2, dadurch gekennzeichnet, daß man Di-(3-Deoxy-D-Glyceropentulose)-Dianhydrid (Formel II, R = H) herstellt.

7. Verfahren nach Anspruch 2, dadurch gekennzeichnet, daß man Diacetyl-Di-(3-Deoxy-D-Glyceropentulose)-Dianhydrid (Formel II, $R = COCH_3$) herstellt.

**Patentansprüche für folgenden Vertragsstaat : GR**

1. Verwendung einer oder mehrerer Verbindungen der allgemeinen Formel I und/oder II

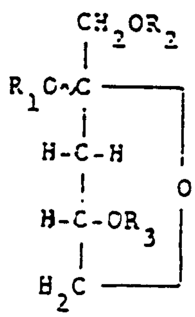

(I)

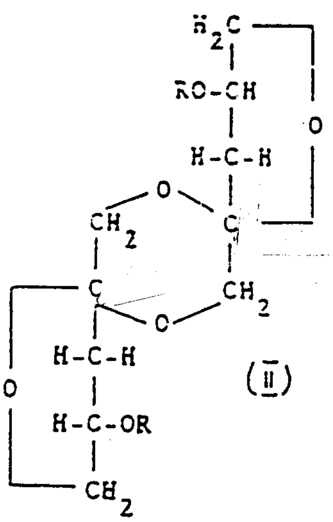

(II)

worin $R_1$ ein Wasserstoffatom oder eine Alkylgruppe oder Acylgruppe mit je 1 bis 7 Kohlenstoffatomen bedeutet, $R_2$ und $R_3$ ein Wasserstoffatom oder, wenn $R_1$ eine Alkylgruppe ist, auch eine Acylgruppe $R_4CO$ bedeuten, R ein Wasserstoffatom oder eine Acylgruppe $R_5CO$ bedeutet, und $R_4$ und $R_5$ eine Alkylgruppe mit 1 bis 7 Kohlenstoffatomen bedeuten, und übliche pharmazeutische Träger- und/oder Verdünnungsmittel enthält
zur Herstellung eines anti-proliferativen cytotoxischen Arzneimittels.

2. Verfahren zur Herstellung der Wirkstoffe der Formel I und II nach Anspruch 1, worin $R_1$, $R_2$, $R_3$, $R_4$, $R_5$ und R die in Anspruch 1 angegebenen Bedeutungen besitzen,
**dadurch gekennzeichnet,**
daß man $\alpha$- oder $\beta$-1,4-verknüpfte Di- oder Oligosaccharide, die an ihrem reduzierenden Ende ein Glucose-, Fructose- oder Mannose-Molekül enthalten, mit anorganischen oder organischen Basen behandelt, aus der erhaltenen Reaktionsmischung die 3-Deoxy-D-Glyceropentulose (3-DGP) der Formel I, worin $R_1$, $R_2$ und $R_3$ ein Wasserstoffatom bedeuten, isoliert, und, wenn erwünscht, das 3-DGP auf an sich bekannte Weise in sein Glycosid (Formel I, $R_1$ = Alkyl), Diacylglycosid (Formel I, $R_1$ = Alkyl, $R_2$, $R_3 = R_4CO$), Dianhydrid (Formel II, R = H) oder Diacyldianhydrid (Formel II, $R = R_5CO$) überführt.

3. Methyl-3-Deoxy-D-Glyceropentulosid (Formel I, $R_1 = CH_3$, $R_2$, $R_3 = H$).

4. n-Butyl-3-Deoxy-D-Glyceropentulosid (Formel I, $R_1 = n\text{-}C_4H_9$, $R_2$, $R_3 = H$).

**5.** n-Butyl-Diacetyl-3-Deoxy-D-Glyceropentulosid (Formel I, $R_1$ = n-$C_4H_9$, $R_2$, $R_3$ = $COCH_3$ ).

**6.** Di-(3-Deoxy-D-Glyceropentulose)-Dianhydrid (Formel II, R = H).

**7.** Diacetyl-Di-(3-Deoxy-D-Glyceropentulose)-Dianhydrid (Formel II, R = $COCH_3$).

**Claims**
**Claims for the following Contracting States : DE, GB, FR, IT, NL, SE, LI, CH, BE, AT, LU**

**1.** A drug, characterized in that it contains as the active ingredient one or more of the compounds of general formulae I and/or II:

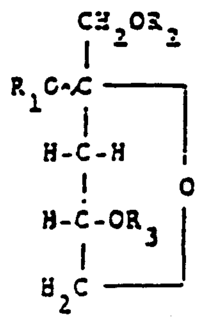

(I)

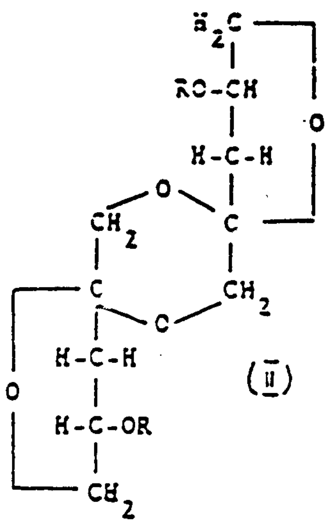

(II)

in which $R_1$ is a hydrogen atom or an alkyl group or acyl group each having 1 to 7 carbon atoms, $R_2$ and $R_3$ are a hydrogen atom or else, if $R_1$ is an alkyl group, are an acyl group $R_4CO$, R is a hydrogen atom or an acyl group $R_5CO$, and $R_4$ and $R_5$ are an alkyl group having 1 to 7 carbon atoms, and conventional pharmaceutical excipients and/or diluents.

**2.** A process for the preparation of the active ingredients of formulae I and II according to Claim 1, in which $R_1$, $R_2$, $R_3$, $R_4$, $R_5$ and R are as defined in Claim 1, characterized in that $\alpha$- or $\beta$-1,4-linked di- or oligosaccharides containing a glucose, fructose or mannose molecule at their reducing end are treated with inorganic or organic bases, the 3-deoxy-D-glyceropentulose (3-DGP) of formula I in which $R_1$, $R_2$ and $R_3$ are a hydrogen atom is isolated from the resulting reaction mixture and, if desired, the 3-DGP is converted in a manner known per se to its glycoside (formula I, $R_1$ = alkyl), diacylglycoside (formula I, $R_1$ = alkyl, $R_2$, $R_3$ = $R_4CO$), dianhydride (formula II, R = H) or diacyldianhydride (formula II, R = $R_5CO$).

**3.** Methyl-3-deoxy-D-glyceropentuloside (formula I, $R_1$ = $CH_3$, $R_2$, $R_3$ = H).

**4.** n-Butyl-3-deoxy-D-glyceropentuloside (formula I, $R_1$ = n-$C_4H_9$, $R_2$, $R_3$ = H).

**5.** n-Butyl-diacetyl-3-deoxy-D-glyceropentuloside (formula I, $R_1$ = n-$C_4H_9$, $R_2$, $R_3$ = $COCH_3$).

**6.** Di(3-deoxy-D-glyceropentulose) dianhydride (formula II, R = H).

**7.** Diacetyldi(3-deoxy-D-glyceropentulose) dianhydride (formula II, R = $COCH_3$).

**8.** Use of one or more compounds of general formulae I and/or II for the preparation of an antiproliferative/cytotoxic drug.

**Claims for the following Contracting State : ES**

1. Use of one or more compounds of general formulae I and/or II:

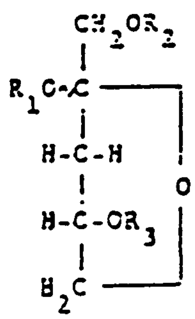

(I)

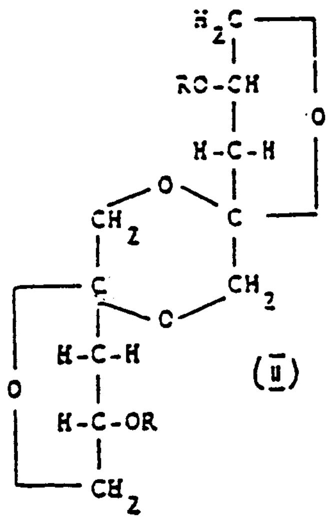

(II)

in which $R_1$ is a hydrogen atom or an alkyl group or acyl group each having 1 to 7 carbon atoms, $R_2$ and $R_3$ are a hydrogen atom or else, if $R_1$ is an alkyl group, are an acyl group $R_4CO$, R is a hydrogen atom or an acyl group $R_5CO$, and $R_4$ and $R_5$ are an alkyl group having 1 to 7 carbon atoms, and contains conventional pharmaceutical excipients and/or diluents, for the preparation of an antiproliferative cytotoxic drug.

2. A process for the preparation of the active ingredients of formulae I and II according to Claim 1, in which $R_1$, $R_2$, $R_3$, $R_4$, $R_5$ and R are as defined in Claim 1, characterized in that $\alpha$- or $\beta$-1,4-linked di- or oligosaccharides containing a glucose, fructose or mannose molecule at their reducing end are treated with inorganic or organic bases, the 3-deoxy-D-glyceropentulose (3-DGP) of formula I in which $R_1$, $R_2$ and $R_3$ are a hydrogen atom is isolated from the resulting reaction mixture and, if desired, the 3-DGP is converted in a manner known per se to its glycoside (formula I, $R_1$ = alkyl), diacylglycoside (formula I, $R_1$ = alkyl, $R_2$, $R_3$ = $R_4CO$), dianhydride (formula II, R = H) or diacyldianhydride (formula II, R = $R_5CO$).

3. A process according to Claim 2, characterized in that methyl-3-deoxy-D-glyceropentuloside (formula I, $R_1$ = $CH_3$, $R_2$, $R_3$ = H) is prepared.

4. A process according to Claim 2, characterized in that n-butyl-3-deoxy-D-glyceropentuloside (formula I, $R_1$ = n-$C_4H_9$, $R_2$, $R_3$ = H) is prepared.

5. A process according to Claim 2, characterized in that n-butyl-diacetyl-3-deoxy-D-glyceropentuloside (formula I, $R_1$ = n-$C_4H_9$, $R_2$, $R_3$ = $COCH_3$) is prepared.

6. A process according to Claim 2, characterized in that di(3-deoxy-D-glyceropentulose) dianhydride (formula II, R = H) is prepared.

7. A process according to Claim 2, characterized in that diacetyldi(3-deoxy-D-glyceropentulose) dianhydride (formula II, R = $COCH_3$) is prepared.

**Claims for the following Contracting State : GR**

**1.** Use of one or more compounds of general formulae I and/or II:

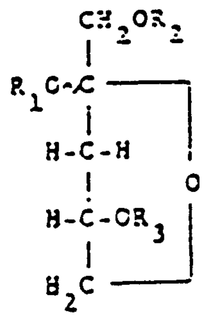

(I)

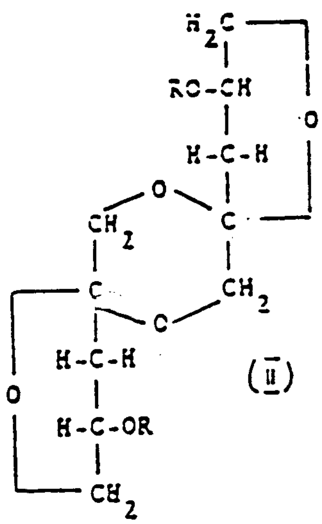

(II)

in which $R_1$ is a hydrogen atom or an alkyl group or acyl group each having 1 to 7 carbon atoms, $R_2$ and $R_3$ are a hydrogen atom or else, if $R_1$ is an alkyl group, are an acyl group $R_4CO$, R is a hydrogen atom or an acyl group $R_5CO$, and $R_4$ and $R_5$ are an alkyl group having 1 to 7 carbon atoms, and contains conventional pharmaceutical excipients and/or diluents (sic), for the preparation of an antiproliferative cytotoxic drug.

**2.** A process for the preparation of the active ingredients of formulae I and II according to Claim 1, in which $R_1$, $R_2$, $R_3$, $R_4$, $R_5$ and R are as defined in Claim 1, characterized in that $\alpha$- or $\beta$-1,4-linked di- or oligosaccharides containing a glucose, fructose or mannose molecule at their reducing end are treated with inorganic or organic bases, the 3-deoxy-D-glyceropentulose (3-DGP) of formula I in which $R_1$, $R_2$ and $R_3$ are a hydrogen atom is isolated from the resulting reaction mixture and, if desired, the 3-DGP is converted in a manner known per se to its glycoside (formula I $R_1$ = alkyl), diacylglycoside (formula I, $R_1$ = alkyl, $R_2$, $R_3$ = $R_4CO$), dianhydride (formula II, R = H) or diacyldianhydride (formula II, R = $R_5CO$).

**3.** Methyl-3-deoxy-D-glyceropentuloside (formula I, $R_1$ = $CH_3$, $R_2$, $R_3$ = H).

**4.** n-Butyl-3-deoxy-D-glyceropentuloside (formula I, $R_1$ = n-$C_4H_9$, $R_2$, $R_3$ = H).

**5.** n-Butyl-diacetyl-3-deoxy-D-glyceropentuloside (formula I, $R_1$ = n-$C_4H_9$, $R_2$, $R_3$ = $COCH_3$).

**6.** Di(3-deoxy-D-glyceropentulose) dianhydride (formula II, R = H).

**7.** Diacetyldi(3-deoxy-D-glyceropentulose) dianhydride (formula II, R = $COCH_3$).

**Revendications**

**Revendications pour les Etats contractants suivants : DE, GB, FR, IT, NL, SE, LI, CH, BE, AT, LU**

**1.** Médicament, caractérisé en ce qu'il contient comme substance active un ou plusieurs composés de formule générale I et/ou II

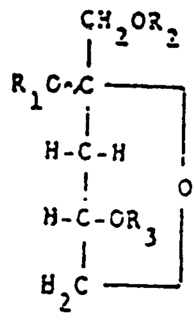

(I)

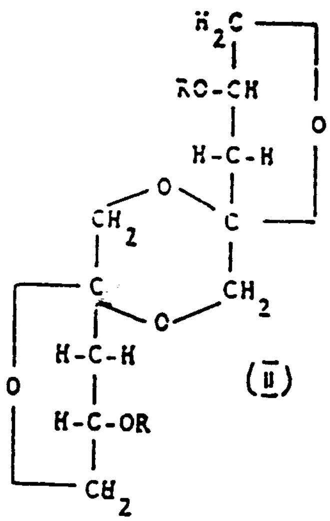

(II)

dans lesquelles $R_1$ représente un atome d'hydrogène ou un coupe alkyle ou acyle ayant chacun 1 à 7 atomes de carbone, $R_2$ et $R_3$ représentent un atome d'hydrogène ou encore, lorsque $R_1$ est un groupe alkyle, un groupe acyle $R_4CO$, R représente un atome d'hydrogène ou un groupe acyle $R_5CO$, et $R_4$ et $R_5$ représentent un groupe alkyle de 1 à 7 atomes de carbone, et des supports et/ou diluants pharmaceutiques classiques.

**2.** Procédé de préparation des substances actives de formule I et II selon la revendication 1, dans lesquelles $R_1$, $R_2$, $R_3$, $R_4$, $R_5$ et R ont les significations données dans la revendication 1, caractérisé en ce que l'on traite des di- ou oligosaccharides à liaisons $\alpha$- ou $\beta$-1,4, qui contiennent à leur extrémité réductrice une molécule de glucose, de fructose ou de mannose, avec des bases inorganiques ou organiques, on isole à partir du mélange réactionnel obtenu le 3-désoxy-D-glycéropentulose (3-DGP) de formule I dans laquelle $R_1$, $R_2$ et $R_3$ représentent un atome d'hydrogène et, si désiré, on transforme de façon connue en soi le 3-DGP en son glycoside (formule I, $R_1$ = alkyle), diacylglycoside (formule I, $R_1$ = alkyle, $R_2$, $R_3$ = $R_4CO$), dianhydride (formule II, R = H) ou diacyldianhydride (formule II, R = $R_5CO$).

**3.** 3-désoxy-D-glycéropentuloside de méthyle (formule I, $R_1$ = $CH_3$, $R_2$, $R_3$ = H).

**4.** 3-désoxy-D-glycéropentuloside de n-butyle (formule I, $R_1$ = n-$C_4H_9$, $R_2$, $R_3$ = H).

**5.** Diacétyl-3-désoxy-D-glycéropentuloside de n-butyle (formule I, $R_1$ = n-$C_4H_9$, $R_2$, $R_3$ = $COCH_3$).

**6.** Dianhydride de di(3-désoxy-D-glycéropentulose) (formule II, R = H).

**7.** Dianhydride de diacétyldi(3-désoxy-D-glycéropentulose) (formule II, R = $COCH_3$).

**8.** Utilisation d'un ou plusieurs dérivés de formule générale I et/ou II pour la préparation d'un médicament anti-prolifération/cytotoxique.

**Revendications pour l'Etat contractant suivant : ES**

1. Utilisation d'un ou plusieurs composés de formule générale I et/ou II

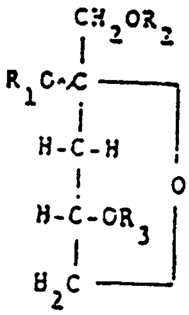

(I)

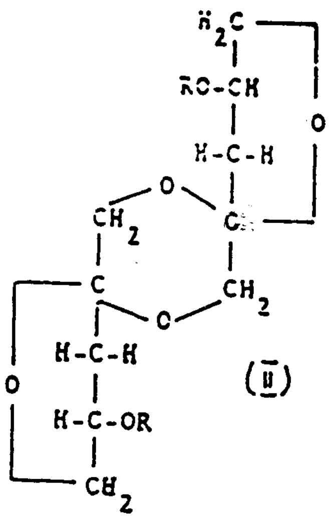

(II)

dans lesquelles $R_1$ représente un atome d'hydrogène ou un groupe alkyle ou acyle ayant chacun 1 à 7 atomes de carbone, $R_2$ et $R_3$ représentent un atome d'hydrogène ou encore, lorsque $R_1$ est un groupe alkyle, un groupe acyle $R_4CO$, R représente un atome d'hydrogène ou un groupe acyle $R_5CO$, et $R_4$ et $R_5$ représentent un groupe alkyle de 1 à 7 atomes de carbone, et de supports et/ou diluants pharmaceutiques classiques, pour la préparation d'un médicament cytotoxique anti-prolifération.

2. Procédé de préparation des substances actives de formule I et II selon la revendication 1, dans lesquelles $R_1$, $R_2$, $R_3$, $R_4$, $R_5$ et R ont les significations données dans la revendication 1, caractérisé en ce que l'on traite des di- ou oligosaccharides à liaisons $\alpha$- ou $\beta$-1,4, qui contiennent à leur extrémité réductrice une molécule de glucose, de fructose ou de mannose, avec des bases inorganiques ou organiques, on isole à partir du mélange réactionnel obtenu le 3-désoxy-D-glycéropentulose (3-DGP) de formule I dans laquelle $R_1$, $R_2$ et $R_3$ représentent un atome d'hydrogène et, si désiré, on transforme de façon connue en soi le 3-DGP en son glycoside (formule I, $R_1$ = alkyle), diacylglycoside (formule I, $R_1$ = alkyle, $R_2$, $R_3$ = $R_4CO$), dianhydride (formule II, R = H) ou diacyldianhydride (formule II, R = $R_5CO$).

3. Procédé selon la revendication 2, caractérisé en ce que l'on prépare le 3-désoxy-D-glycéropentuloside de méthyle (formule I, $R_1$ = $CH_3$, $R_2$, $R_3$ = H).

4. Procédé selon la revendication 2, caractérisé en ce que l'on prépare le 3-désoxy-D-glycéropentuloside de n-butyle (formule I, $R_1$ = n-$C_4H_9$, $R_2$, $R_3$ = H).

5. Procédé selon la revendication 2, caractérisé en ce que l'on prépare le diacétyl-3-désoxy-D-glycéropentuloside de n-butyle (formule I, $R_1$ = n-$C_4H_9$, $R_2$, $R_3$ = $COCH_3$).

6. Procédé selon la revendication 2, caractérisé en ce que l'on prépare le dianhydride de di(3-désoxy-D-glycéropentulose) (formule II, R = H).

7. Procédé selon la revendication 2, caractérisé en ce que l'on prépare le dianhydride de diacétyldi(3-désoxy-D-glycéropentulose) (formule II, R = $COCH_3$).

**Revendications pour l'Etat contractant suivant : GR**

1. Utilisation d'un ou plusieurs composés de formule générale I et/ou II

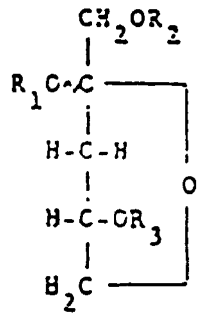

(I)

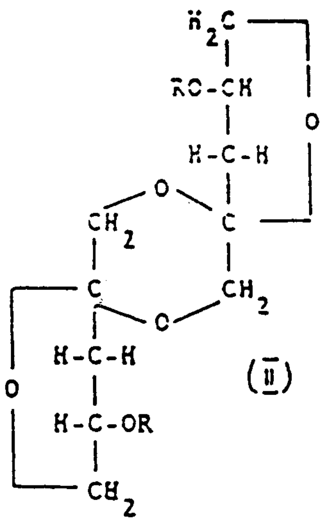

(II)

dans lesquelles $R_1$ représente un atome d'hydrogène ou un groupe alkyle ou acyle ayant chacun 1 à 7 atomes de carbone, $R_2$ et $R_3$ représentent un atome d'hydrogène ou encore, lorsque $R_1$ est un groupe alkyle, un groupe acyle $R_4CO$, R représente un atome d'hydrogène ou un groupe acyle $R_5CO$, et $R_4$ et $R_5$ représentent un groupe alkyle de 1 à 7 atomes de carbone, et de supports et/ou diluants pharmaceutiques classiques, pour la préparation d'un médicament cytotoxique anti-prolifération.

2. Procédé de préparation des substances actives de formule I et II selon la revendication 1, dans lesquelles $R_1$, $R_2$, $R_3$, $R_4$, $R_5$ et R ont les significations données dans la revendication 1, caractérisé en ce que l'on traite des di- ou oligosaccharides à liaisons $\alpha$- ou $\beta$-1,4, qui contiennent à leur extrémité réductrice une molécule de glucose, de fructose ou de mannose, avec des bases inorganiques ou organiques, on isole à partir du mélange réactionnel obtenu le 3-désoxy-D-glycéropentulose (3-DGP) de formule I dans laquelle $R_1$, $R_2$ et $R_3$ représentent un atome d'hydrogène et, si désiré, on transforme de façon connue en soi le 3-DGP en son glycoside (formule I, $R_1$ = alkyle), diacylglycoside (formule I, $R_1$ = alkyle, $R_2$, $R_3$ = $R_4CO$), dianhydride (formule II, R = H) ou diacyldianhydride (formule II, R = $R_5CO$).

3. 3-désoxy-D-glycéropentuloside de méthyle (formule I, $R_1$ = $CH_3$, $R_2$, $R_3$ = H).

4. 3-désoxy-D-glycéropentuloside de n-butyle (formule I, $R_1$ = n-$C_4H_9$, $R_2$, $R_3$ = H).

5. Diacétyl-3-désoxy-D-glycéropentuloside de n-butyle (formule I, $R_1$ = n-$C_4H_9$, $R_2$, $R_3$ = $COCH_3$).

6. Dianhydride de di(3-désoxy-D-glycéropentulose) (formule II, R = H).

7. Dianhydride de diacétyldi(3-désoxy-D-glycéropentulose) (formule II, R = $COCH_3$).

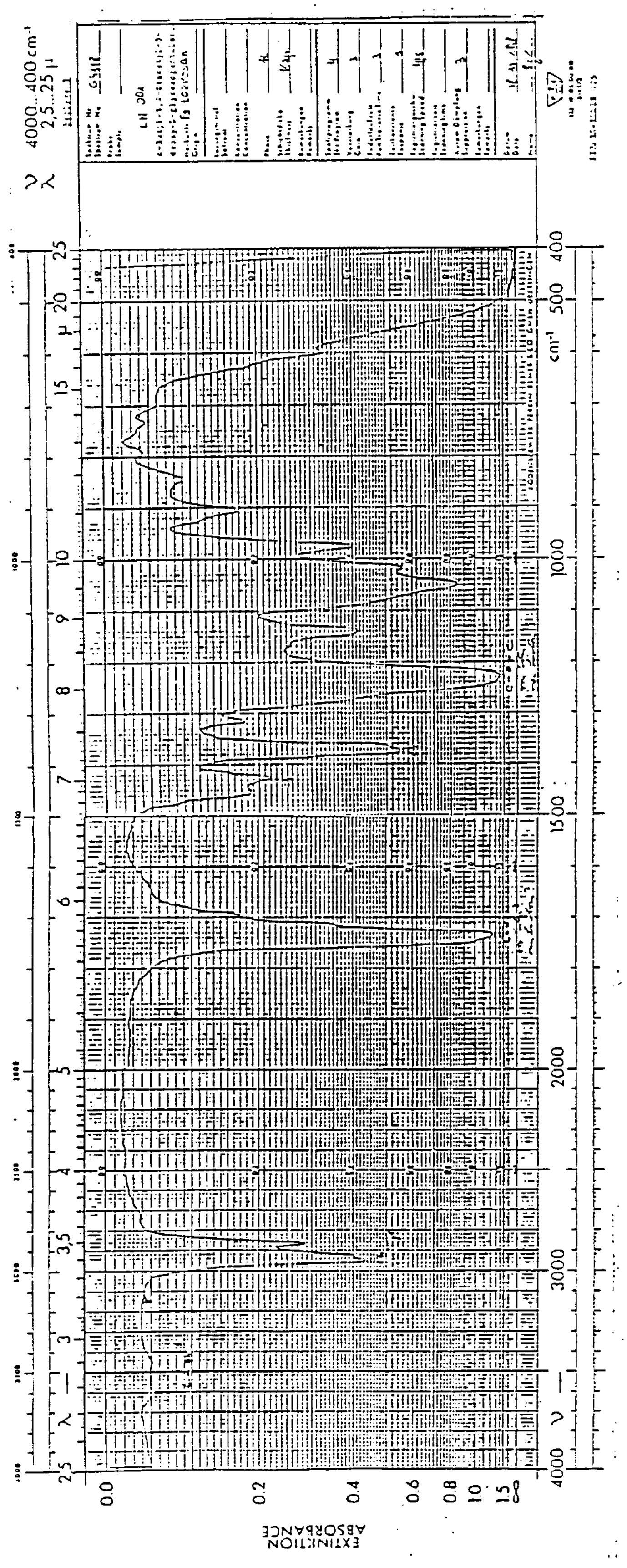


Fig. 1

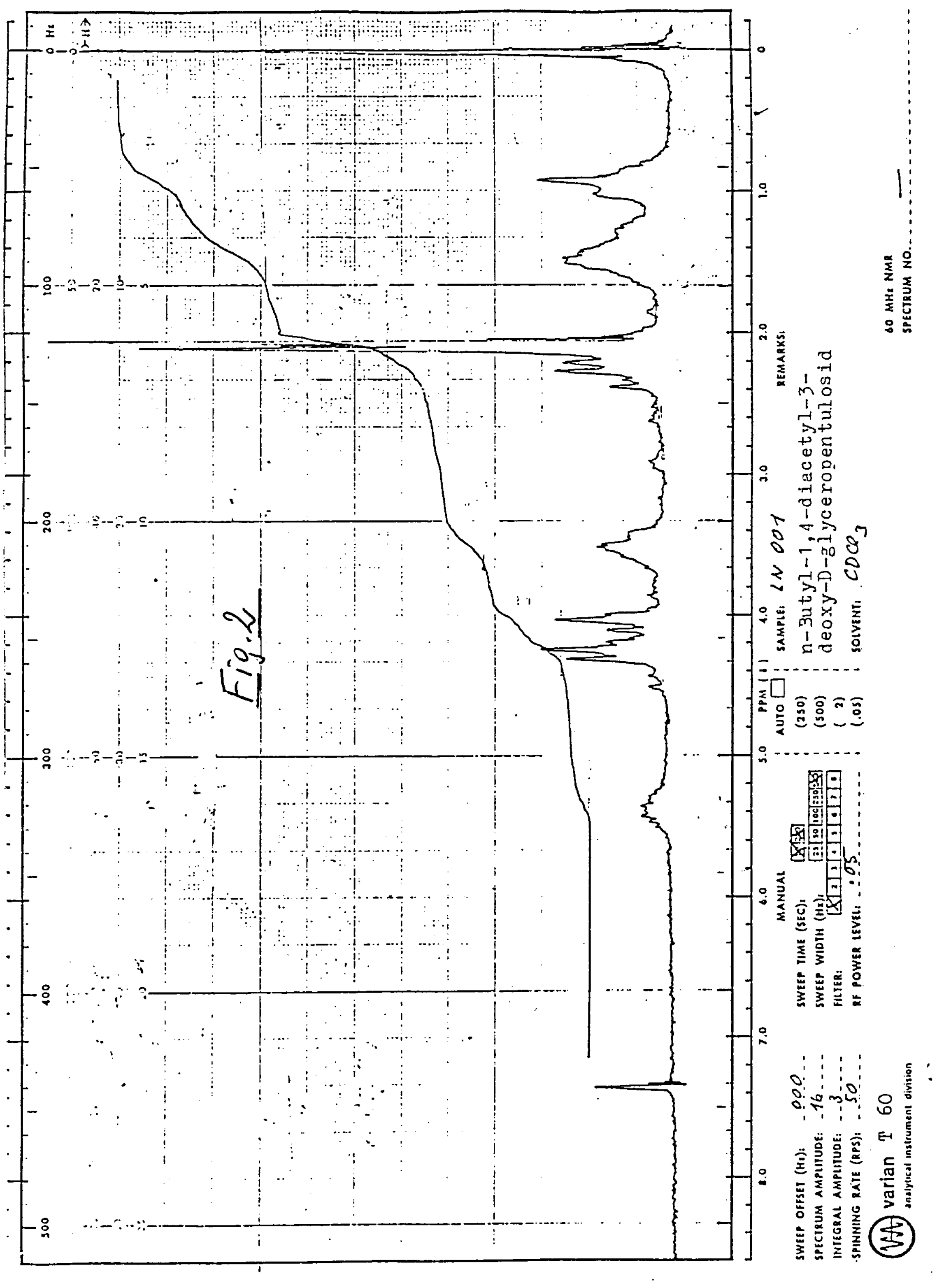
Fig.2
n-Butyl-1,4-diacetyl-3-deoxy-D-glyceropentulosid
60 MHz NMR
varian T 60

Fig.3

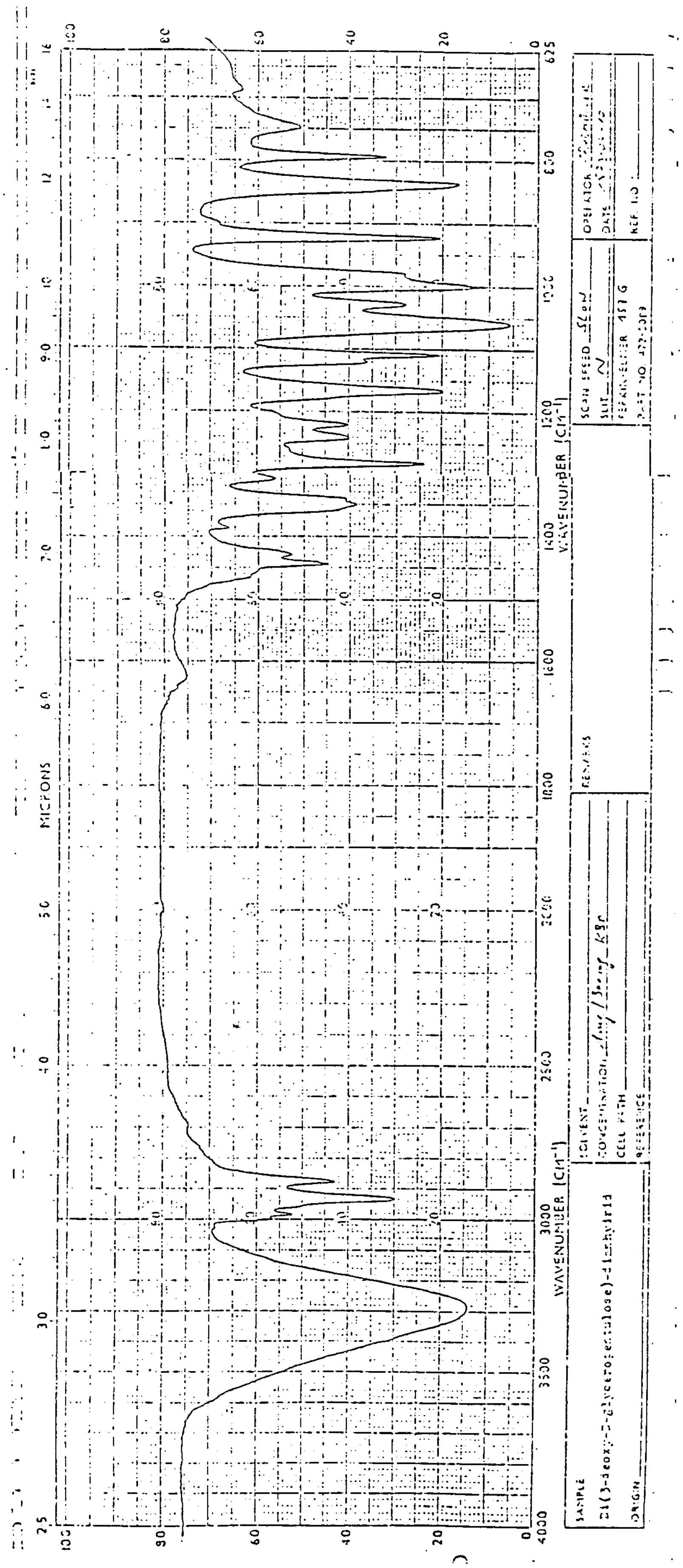

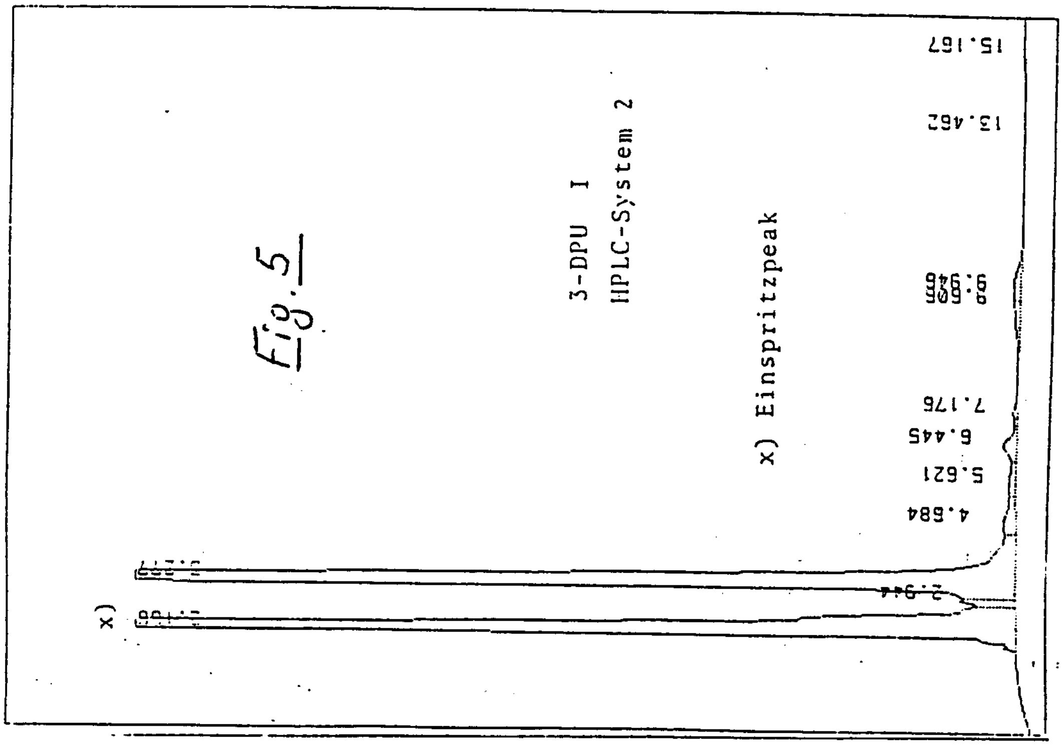
3-DPU I
HPLC-System 2
x) Einspritzpeak
x)
15.167
13.462
7.176
6.445
5.621
4.584
2.944

Fig. 5

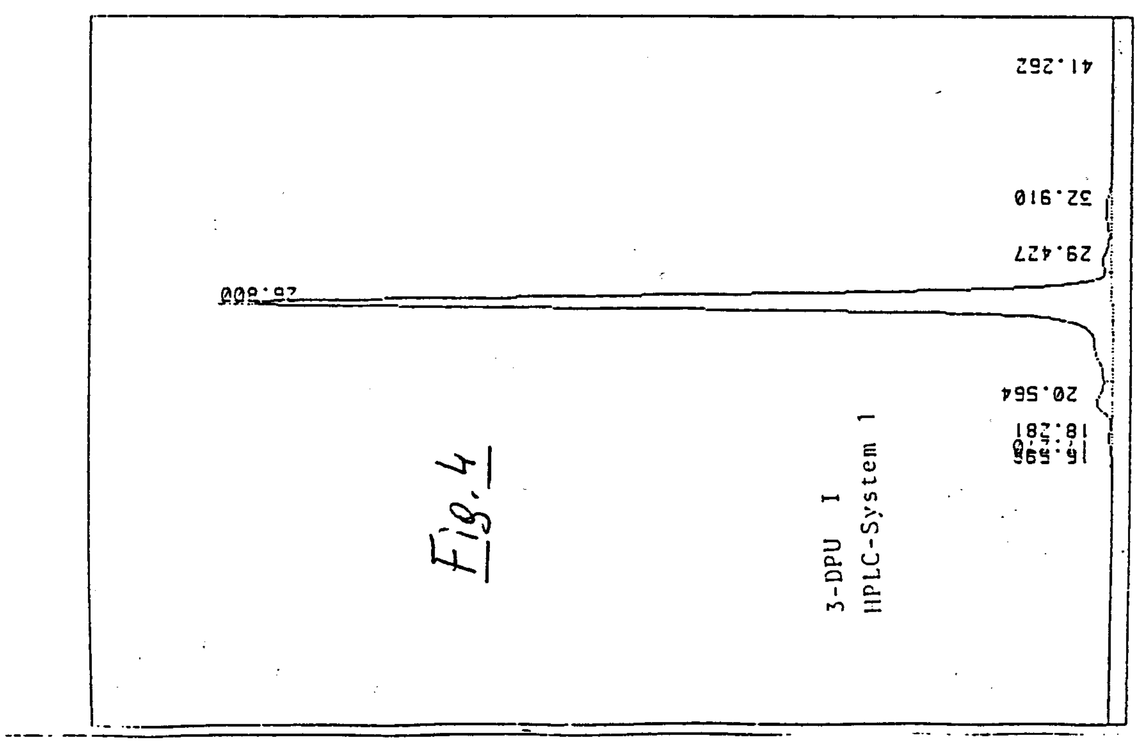
3-DPU I
HPLC-System 1
41.262
32.910
29.427
20.564
18.281

Fig. 4

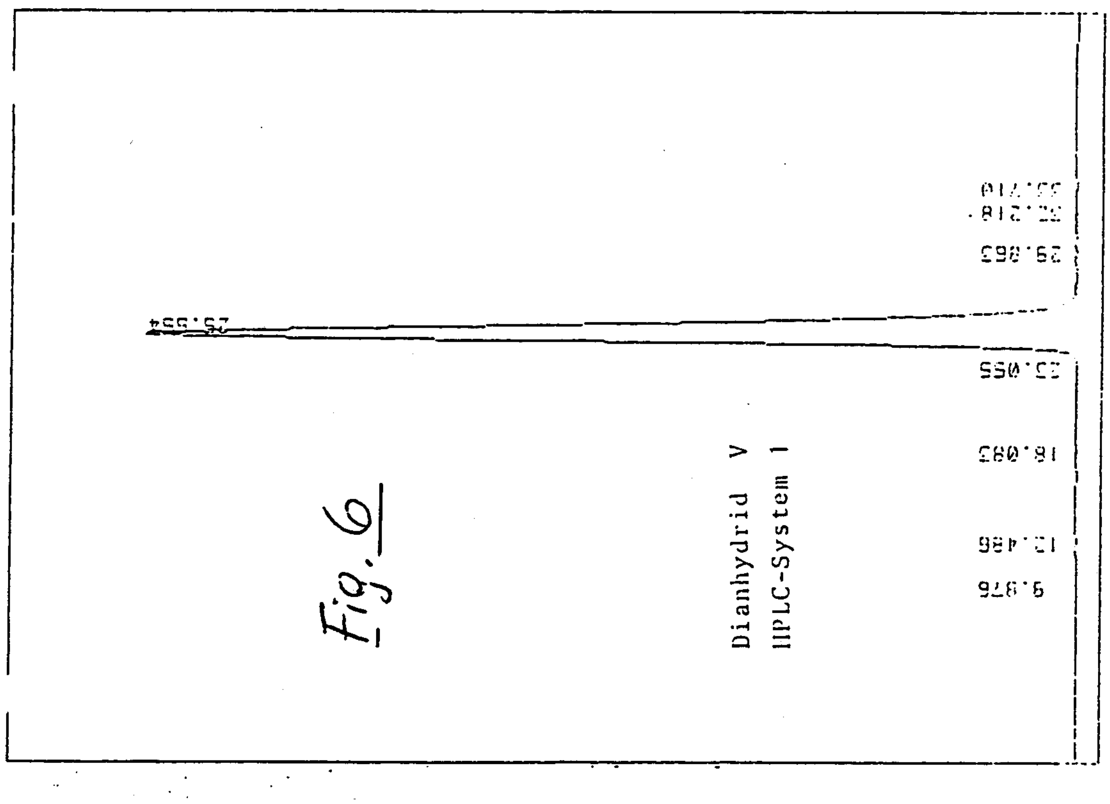
Fig. 6
Dianhydrid V
HPLC-System 1
9.876
12.486
18.083
23.055
25.554
29.863
32.218